Europäisches Patentamt

European Patent Office

Office européen des brevets

(19)

(11) Numéro de publication: **0 109 913**
**A2**

## (12) DEMANDE DE BREVET EUROPEEN

(21) Numéro de dépôt: 83440046.7

(22) Date de dépôt: 07.09.83

(51) Int. Cl.³: **A 61 M 5/315**

(30) Priorité: 03.11.82 FR 8218546
03.08.83 FR 8312932

(43) Date de publication de la demande:
30.05.84 Bulletin 84/22

(84) Etats contractants désignés:
CH DE GB IT LI SE

(71) Demandeur: MICRO-MEGA
5-12 rue du Tunnel
F-25000 Besancon(FR)

(72) Inventeur: Leonard, Henri
4 rue Jean de Vienne
F-25000 Besancon(FR)

(72) Inventeur: Seigneurin, Michel
St-Cergues
F-74140 Douvaine(FR)

(74) Mandataire: Bossard, Jacques-René
CABINET ARBOUSSE BASTIDE 20 rue de Copenhague
F-67000 Strasbourg(FR)

(54) Seringue dentaire pour injections intra-ligamentaires.

(57) Seringue dentaire pour injections intra-ligamentaires, du type comportant une aiguille d'injection, un conteneur contenant le produit à injecter et un piston commandant le déplacement du fond du conteneur. Le déplacement du piston (12) vers le conteneur est commandé par un levier (3) comportant un cliquet (19) coopérant avec une crémaillère (15) ménagée sur le piston, assurant une démultiplication de la pression manuelle exercée par l'utilisateur sur ledit levier.

FIG. 1

EP 0 109 913 A2

–1–

# SERINGUE DENTAIRE A MULTIPLICATION DE PRESSION.

La présente invention a pour objet une seringue dentaire pour injections intra-ligamentaires, du type comportant généralement une aiguille d'injection, un conteneur contenant le produit à injecter et un piston commandant le déplacement du fond du conteneur.

Des seringues de ce type ont été déjà décrites par exemple dans le brevet FR 1 583 163 au nom de la demanderesse.

Toutefois, de telles seringues ne peuvent convenir telles quelles pour l'injection de produits dans les ligaments situés entre l'os de la machoire et la dent.

En effet, l'intervalle entre l'os et la dent est étroit, et on doit y introduire une aiguille très fine et souple. Pour que le produit arrive à passer dans cette aiguille et puisse être expulsé, il faut donc lui imprimer une pression d'injection très élevée.

Une simple pression sur le piston imprimée par la paume de la main, comme cela est le cas pour les seringues ordinaires, ne peut convenir.

L'invention a pour objet en conséquence une seringue pour injections intra-ligamentaires permettant de remédier à ces inconvénients.

-2-

Conformément à l'invention, ce résultat est obtenu avec une seringue dentaire pour injections intra-ligamentaires, du type comportant une aiguille d'injection, un conteneur contenant le produit à injecter et un piston commandant le déplacement du fond du conteneur, caractérisé en ce que le déplacement du piston vers le conteneur est commandé par un levier comportant un cliquet coopérant avec une crémaillère ménagée sur le piston, assurant une démultiplication de la pression manuelle exercée par l'utilisateur sur ledit levier.

La force de la pression manuelle exercée par l'utilisateur étant multipliée, l'expulsion du produit hors du conteneur peut intervenir sans problèmes.

En outre l'effort exercé par l'utilisateur étant malgré tout moindre qu'avec les seringues de l'art antérieur, il s'ensuit de meilleures conditions de travail et une meilleure précision dans l'intervention.

Selon un premier mode de réalisation de l'invention, le produit à injecter est un liquide, qui est contenu dans une carpule telle que décrite dans le brevet antérieur précité, à savoir en forme de petit réservoir oblong, dont l'extrémité postérieure consiste en un disque mobile, et dont l'extrémité antérieure est obturée par une pastille de caoutchouc destinée à êter perforée par l'aiguille d'injection.

Dans ce mode de réalisation, l'invention vise au surplus l'adjonction entre l'extrémité antérieure du porte-carpule et la carpule, d'une pièce en forme de manchon de protection du bouchon de la carpule contre les déchirures accidentelles pouvant résulter de la déformation de ce bouchon sous la pression du liquide qu'elle contient. Selon l'invention ce manchon vient entourer la portion postérieure de l'aiguille en s'épanouissant à son extrémité arrière pour s'adapter à la forme du porte-carpule, la partie centrale de cette extrémité présentant un relief repoussant légèrement le bouchon en caoutchouc de la carpule au niveau de l'extrémité de l'aiguille.

0109913

-3-

Selon un second mode de réalisation, le produit à injecter est une pâte, et dans ce cas, au lieu de la carpule qui vient d'être décrite, l'invention prévoit un conteneur de pâte se terminant à l'avant par une canule d'injection, éventuellement coudée, et à l'arrière par un bouchon élastique subissant l'action d'un piston auxiliaire mobile dans une pièce servant de porte-conteneur, et subissant lui-même l'action du piston principal contenu dans le corps de la seringue, et actionné par les moyens décrits ci-dessus. Un ressort de rappel tend à éloigner ce piston auxiliaire de ce bouchon, la course de ce piston auxiliaire étant délimitée par deux butées logées dans le porte-conteneur.

Dans le premier mode de réalisation, la carpule est introduite par l'arrière du porte-conteneur. Dans le second, le piston auxiliaire interdisant cette introduction du conteneur par l'arrière, la mise en place du conteneur s'effectue par une lumière pratiquée dans la partie antérieure du porte-conteneur.

On comprendra mieux l'invention à l'aide de la description ci-après, faite en référence aux dessins annexés dans lesquels :

- la figure 1 est une vue en coupe longitudinale partielle d'une seringue conforme au premier mode de réalisation de l'invention ;

- la figure 2 est une variante de la figure 1 avec un support d'aiguille incliné ;

- la figure 3 est une variante d'exécution du dispositif démultiplicateur de la seringue de la figure 1, en coupe longitudinale et en vue partielle.

- la figure 4 représente le manchon de protection de la carpule, l'aiguille étant en place.

- la figure 5 est un schéma à grande échelle montrant la forme du bouchon de la carpule.

- la figure 6 est une vue latérale avec arrachement partiel du porte-conteneur destiné à la variante de l'inven-

-4-

tion destinée à l'injection de produits pâteux.

- la figure 7 est une vue analogue à la figure 6, dans le cas où la canule d'injection est coudée.

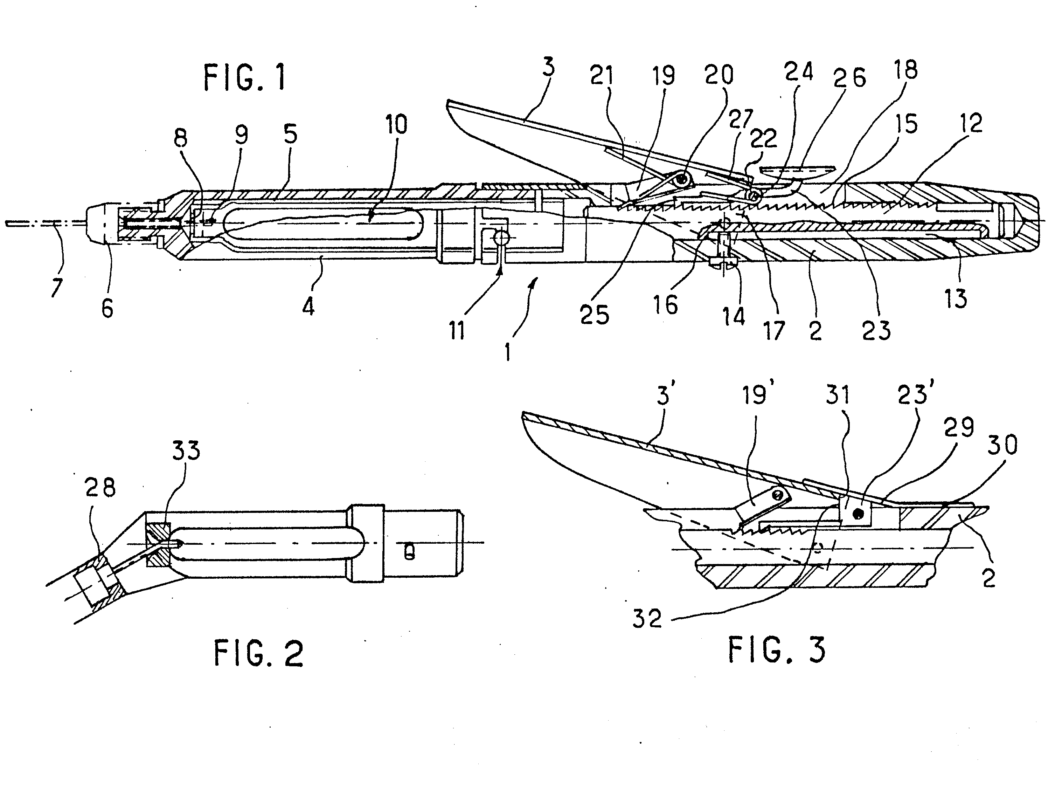

On va se référer d'abord aux figures 1 à 3, c'est-à-dire au mode de réalisation de l'invention dans lequel la seringue est destinée à l'injection intra-ligamentaire de produits liquides.

Dans ce cas la seringue (1), telle que représentée dans son entier à la figure 1 comporte essentiellement :

- un corps (2) sur lequel est articulé le levier (3) de commande de la seringue,

- un support de carpule (4) dans lequel est enfilée la carpule (5),

- un embout (6) comportant une aiguille d'injection (7).

La carpule (5) se présente, de manière connue, sous la forme d'un réservoir oblong renfermant le liquide à injecter. Elle comporte à l'une de ses extrémités une pastille de caoutchouc (8) qui sera traversée par la partie arrière (9) de l'aiguille (7). De ce fait, le contenu de la carpule peut n'être utilisé que partiellement. Le fond de la carpule consiste en un disque mobile en translation et repoussant, dans son déplacement, le liquide à injecter vers l'aiguille.

Le support de carpule (4) comporte une lumière (10) permettant de surveiller le niveau de remplissage de la carpule.

Le support de carpule (4) est relié au corps (2) de manière amovible, pour permettre le placement et le retrait des carpules, par exemple par un système à baïonnette (11), de structure classique et qui ne sera donc pas décrit plus en détails.

Dans le corps (2) coulisse un piston (12) cylindrique, comportant une rainure de guidage longitudinale (13) qui coopère avec une clavette (14) fixe solidaire du corps (2). De cette manière le piston ne peut tourner autour de son axe longitudinal.

Sur une section du piston (12) parallèle à une génératrice est taillée une crémaillère (15), à denture dirigée vers l'arrière de la seringue.

Sur le corps (2) est articulé, comme indiqué précédemment, un levier de commande (3) de section préférentiellement en U, pouvant être monté ainsi à rotation autour de deux demi-axes(16) fixés de chaque côté du corps (2) dans l'alignement l'un de l'autre. Le levier (3) enveloppe ainsi le corps (2), par ses flasques (17) d'articulation.

Entre le levier (3) et le corps (2), au niveau de la zone de recouvrement dudit corps par le levier, est ménagée une lumière (18) dans le corps.

Sur le levier (3) est monté un cliquet (19) pivotant autour d'un axe (20) fixé sur ledit levier.

Un ressort (21) appuie d'une part sur le cliquet (19) et d'autre part sur l'intérieur du levier (3), en tendant à l'éloigner du corps (2) sur lequel il bute sur un épaulement (22).

D'autre part, un levier (23) est monté pivotant sur un axe (24) fixé dans le corps (2). Par son extrémité (25), le levier (23) s'enclanche sur la crémaillère (15) et à son autre extrémité il comporte un bouton (26) faisant saillie hors du corps (2) et sur lequel l'utilisateur peut exercer une pression manuelle.Un ressort (27) tend à appliquer l'extrémité (25) du levier en prise avec la crémaillère (15), l'autre branche du ressort prenant appui sur la face interne du levier (3).

Si on presse sur le bouton (26), l'extrémité (25) du levier (3) se dégage de la crémaillère et entraîne dans son mouvement le cliquet (19) en le dégageant donc également de la crémaillère.

Le fonctionnement de cette seringue est le suivant. On place pour commencer le piston à la position représentée figure 1. Pour cela on tient la seringue verticalement, l'aiguille en haut, et on presse sur le bouton (26). Le piston, dégagé du cliquet (19) et de l'extrémité (25) du levier, descend au fond du corps (2) sous le seul effet de son poids.

On introduit alors la carpule dans son support et on accroche celui-ci sur le corps par le système à baïonnette (11). L'ensemble est alors prêt à l'utilisation.

A cet effet, en pressant sur le levier (3), en fait par une simple pression répétée du pouce, comme pour un pompage, l'axe (20) se déplace par rapport aux axes (16). Le cliquet (19) pousse alors d'un cran la crémaillère (15), et donc le piston (12) qui à son tour pousse le disque caoutchouc formant le fond de la carpule. La force du doigt pressant sur le levier est donc transmise sur le disque et donc sur le contenu de la carpule avec un coefficient de multiplication, par exemple de l'ordre de 5.

Dès que l'utilisateur relache sa pression sur le levier (3), celui-ci revient en position haute sous l'effet du ressort (21). Pendant cette opération, le levier (23) maintient par son extrémité (25) le piston en position. L'opération peut alors être répétée, le piston progressant d'une distance correspondant au pas de la crémaillère, ou à un multiple de celui-ci, à chaque pression sur le levier (3).

Dans la réalisation de la figure 1, l'aiguille (7) est montée sur le support (6). Le support (6) peut être solidaire du support (4) ou être monté amovible par celui-ci, par exemple par un système à baïonnette.

Dans la variante de la figure 2, il est préférable que le support d'aiguille soit monté amovible.

Dans cette réalisation, l'aiguille est inclinée par rapport à l'axe de la seringue ce qui est plus aisé pour certaines injections.

Dans ce cas, on opère comme suit :

- placement de l'aiguille sur le support de carpule,
- introduction de la carpule dans son support,

-7-

- accrochage du support de carpule sur le corps de la seringue,

- injection.

Pour s'assurer, dans ce cas, que l'aiguille pénètre bien par sa partie arrière dans la carpule, on pourra prévoir dans le support un canon (33) avec perçage conique. L'extrémité de l'aiguille glissant dans le cône de manière à ce que son extrémité arrière (9) se présente toujours sensiblement parallèlement à l'axe longitudinal de la carpule.

Dans la variante de mise en oeuvre de la figure 3, une languette flexible (29) fixée en (30) sur le corps (2) appuie d'une part sur le dos du levier (23'), de fonction identique au levier (23), et d'autre part sur le dos du levier (3'), de fonction identique à celle du levier (3), les maintenant, ainsi que le cliquet, en position.

Lorsque l'utilisateur presse sur le levier (3'), le piston avance, par le même mécanisme que celui décrit précédemment. Dès que l'on relache la pression, le ressort (29) rappelle le levier (3') en position haute.

Pour dégager le cliquet (19') et le levier (23') de la crémaillère du piston, il suffit de tirer sur le levier (3') au delà de sa position d'équilibre déterminée par la position de repos de la languette (29). La force (31) du levier pousse alors la face (32) du levier (23') et le fait basculer en le dégageant de la crémaillère.

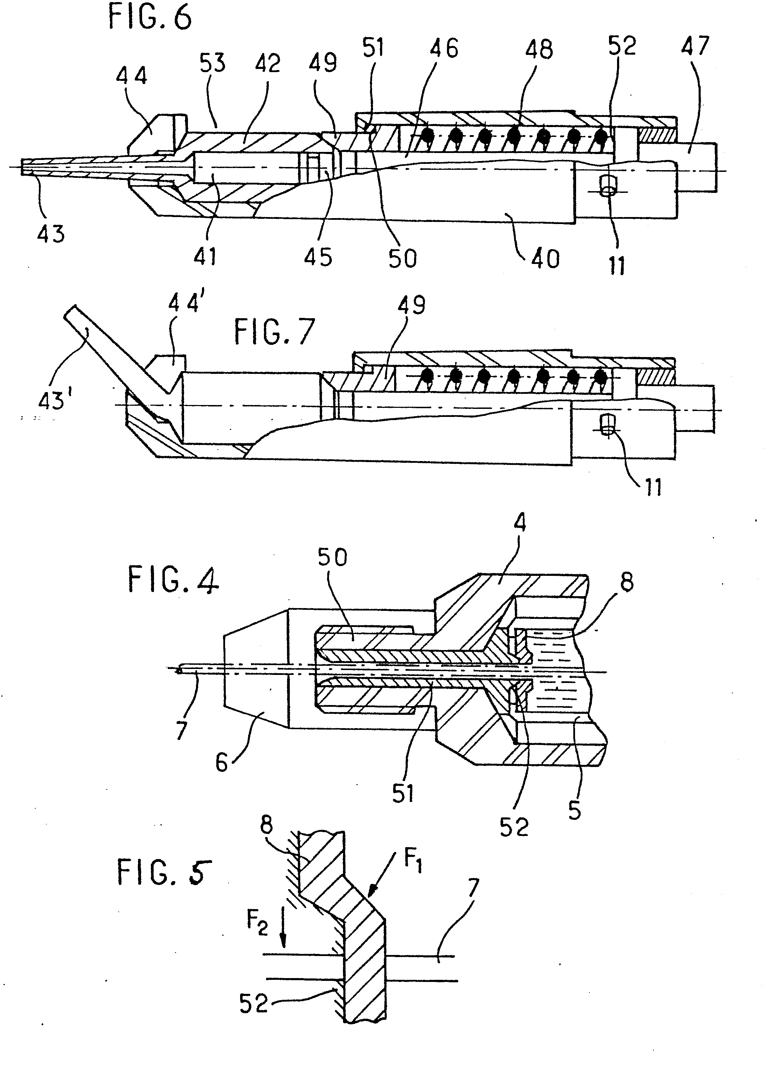

Si l'on se réfère maintenant aux figures 4 et 5, on voit que dans la portion antérieure (50) du porte-carpule (4) est disposé un manchon (51) qui entoure l'aiguille (7) depuis le fond de l'embout (6) jusqu'au bouchon de caoutchouc (8) de la carpule (5). Comme on le voit, le manchon (51) présente à l'arrière une forme évasée, telle qu'il prend appui sur la face avant du porte-carpule (4), tout en possédant un appendice (52) repoussant le bouchon (8) autour de l'aiguille (7).

Cet appendice (52) a pour premier objet d'empêcher ce bouchon de se bomber sous l'influence de la pression du liquide, ce qui risquerait de provoquer une déchirure du bouchon lors de sa pénétration par l'aiguille, surtout dans la version coudée (figure 2).

Au surplus, quand la pression du liquide contenu dans la carpule augmente, au moment de l'utilisation, la résultante des forces de pression s'exerçant sur le bouchon (8) se décompose comme le montre la figure 5 en force $F_1$ s'exerçant obliquement et une force $F_2$, s'exerçant vers l'axe, c'est-à-dire vers l'aiguille. En d'autres termes, le bouchon (8), au moment de l'utilisation, appuie très fortement sur l'aiguille, ce qui contribue à éviter les fuites.

On se réfèrera maintenant à la variante illustrée aux figures 6 et 7 qui représente le mode de réalisation de l'invention dans lequel le produit à injecter est pâteux.

Sur ces figures, on a représenté en (40) le porte-conteneur, analogue au porte-carpule (4) de la réalisation précédente, sauf en ce qui concerne sa portion antérieure. Ce porte-conteneur est de forme générale cylindrique, et constitue un logement dans lequel sont disposés :

- un conteneur prévu pour contenir un produit pâteux, désigné par la référence générale (41) et comprenant un corps principal cylindrique (42), à paroi relativement épaisse, et une canule effilée (43), dépassant à l'extrémité antérieure (44) du porte-conteneur ; ce conteneur est obturé à sa partie postérieure par un bouchon élastique (45) ;

- un piston auxiliaire (46) ayant le même diamètre que le bouchon (45), et dont la partie postérieure (47) fait saillie à l'extérieur du porte-conteneur, de manière à venir au contact du piston principal (12) (non représenté sur ces figures) au moment de l'accrochage dudit porte-conteneur au corps de la seringue par (11). Ce piston auxiliaire est repoussé vers l'arrière par un ressort (48) prenant appui, à l'avant sur une bague (49) logée dans le porte-conteneur et immobilisée contre tout déplacement axial vers l'avant par un épaulement (50) coopérant avec un retard (51) correspondant du porte-conteneur, et à l'arrière sur un épaulement (52) du piston auxiliaire ; le piston est donc, au repos, dans la position représentée sur le dessin.

Le conteneur (42), ne pouvant être introduit dans le porte-conteneur par l'arrière, compte-tenu de la présence du piston auxiliaire (40), est mis en place par une lumière (53) pratiquée longitudinalement dans le porte-conteneur, depuis son extrémité antérieure (44) jusqu'à hauteur du rebord (51). A cet effet, la bague (49) présente un champ biseauté (54) ayant pour rôle à la fois de faciliter cette insertion et de maintenir ensuite le conteneur en place.

Le fonctionnement du dispositif est identique à celui des figures 1 et 2. Le porte-conteneur garni d'un conteneur étant accroché au corps de la seringue par application d'une pression manuelle, l'utilisateur fait avancer pas à pas le piston principal (12), qui transmet son déplacement au piston auxiliaire (46) en opposition au ressort (48). Le piston auxiliaire arrive au contact du bouchon (45), et, poursuivant sa course unidirectionnelle, repousse progressivement ce bouchon dans l'intérieur du conteneur (41). Le bouchon joue alors à son tour le rôle d'un piston vis à vis du produit pâteux qui est progressivement refoulé vers la canule (43) d'où il est lentement expulsé dans sa zone d'utilisation. Par exemple, par une série de pressions sur le levier (3) de la seringue, l'utilisateur pourra, sans effort, refouler un amalgame pâteux dans une cavité dentaire.

La variante de la figure 7 montre une modification de la tête (44') du porte-conteneur, permettant l'utilisation d'une canule (43') oblique par rapport à l'axe général du dispositif.

-10-

REVENDICATIONS.

1.      Seringue dentaire pour injections intra-ligamentaires, du type comportant une aiguille d'injection, un
conteneur contenant le produit à injecter et un piston commandant le déplacement du fond du conteneur, caractérisée en
ce que le déplacement du piston (12) vers le conteneur est
commandé par un levier (3) comportant un cliquet (19) coopérant avec une crémaillère (15) ménagée sur le piston, assurant une démultiplication de la pression manuelle exercée par
l'utilisateur sur ledit levier.

2.      Seringue selon la revendication 1, caractérisée en
ce que le piston cylindrique (12) comporte une rainure de
guidage longitudinale (13) dans laquelle est assujettie une
clavette (14) fixe solidaire du corps (2).

3.      Seringue selon l'une quelconque des revendications
1 et 2, caractérisée en ce que le cliquet (19) est monté
pivotant autour d'un axe (20) fixé sur le levier (3), un
ressort (21) appuyant d'une part sur le cliquet (19) et
d'autre part sur l'intérieur du levier (3), tendant à éloigner ledit levier (3) du corps (2), sur lequel il vient en
butée sur un épaulement (22).

4.      Seringue selon l'une quelconque des revendications
1 à 3, caractérisée en ce qu'un levier (23) est monté pivotant sur un axe (24) fixé dans le corps (2), ledit levier
(23) s'enclanchant par son extrémité (25) sur la crémaillère
(15) et comportant à son autre extrémité un bouton de commande (26), un ressort (27) tendant à appliquer l'extrémité (25)
du levier en prise avec la crémaillère (15), l'autre branche
du ressort prenant appui sur la face interne du levier (3).

5.      Seringue selon la revendication 4, caractérisée en
ce que lorsque l'extrémité (25) du levier (23) se dégage de
la crémaillère, elle entraine avec elle le clique (19).

6.      Seringue selon l'une quelconque des revendications
1 à 3, caractérisée en ce qu'elle comporte une languette flexible (29) fixée sur le corps (2) et appuyant d'une part sur le dos du levier (23') et d'autre part sur le dos du levier (3').

7.      Seringue selon la revendication 6 caractérisée en ce que, lorsque l'on tire le levier (3') au delà de sa position d'équilibre, la face (31) du levier repousse la face (32) du levier (23'), dégageant celui-ci de la crémaillère (15).

8.      Seringue selon l'une quelconque des revendications 1 à 7, caractérisée en ce que l'aiguille (7) est montée sur un support amovible(28) solidarisé du support de conteneur (4) par un système à baïonnette.

10.      Seringue selon l'une quelconque des revendications 1 à 9, caractérisée en ce que l'aiguille (7) est inclinée par rapport à l'axe longitudinal de la seringue.

11.      Seringue selon la revendication 10, caractérisée en ce que la partie arrière (9) de l'aiguille est sensiblement parallèle à l'axe longitudinal de la seringue.

12.      Seringue selon l'une quelconque des revendications 10 et 11, caractérisée en ce que le support comporte un canon (33) avec perçage conique.

13.      Seringue selon l'une quelconque des revendications 1 à 12, caractérisée en ce que le produit à injecter est liquide et contenu dans une carpule (5) introduite dans le porte-conteneur (4) par l'extrémité arrière de celui-ci.

14.      Seringue selon la revendication 13, caractérisée en ce que entre l'extrémité antérieure du porte-carpule (4) et la carpule (5) elle-même, d'une pièce en forme de manchon de protection (51) du bouchon de la carpule contre les déchirures de ce bouchon sous la pression du liquide qu'elle contient.

15.      Seringue selon la revendication 14, caractérisée en ce que ce manchon (51) vient entourer la portion postérieure de l'aiguille en s'épanouissant à son extrémité arrière pour s'adapter à la forme du porte-carpule, la portion centrale (52) de cette extrémité présentant un relief repoussant légèrement le bouchon (8), au niveau de l'extrémité de l'aiguille.

16. Seringue selon l'une quelconque des revendications 1 à 12, caractérisée en ce que, le produit à expulser étant pâteux, il est contenu dans un volume cylindrique (42) se terminant à l'avant par une canule (43) et obturé à l'arrière par un bouchon élastique (45) susceptible d'être repoussé dans l'intérieur du conteneur, un piston auxiliaire (46) étant monté coulissant derrière ce conteneur (42) dans le porte-conteneur, de manière à subir l'action du piston principal (12) de la seringue.

17. Seringue selon la revendication 16, caractérisée en ce que le piston auxiliaire (46) est repoussé vers l'arrière par un ressort de compression (48) prenant appui sur un élément (49) solidaire du porte-conteneur et sur un épaulement (50) du piston.

18. Seringue selon l'une des revendications 16 et 17, caractérisée en ce que le porte-conteneur présente à l'avant une lumière (53) pour l'insertion du conteneur.

19. Seringue selon l'une quelconque des revendications 16 à 18, caractérisée en ce que la canule (43') du conteneur est oblique par rapport à l'axe du dispositif.

FIG. 1

FIG. 2

FIG. 3

0109913

- 1 -

FIG.6

FIG.7

FIG.4

FIG.5